# EUROPEAN PATENT SPECIFICATION

(11) **EP 0 980 863 B1**
(45) Date of publication and mention of the grant of the patent: **02.02.2005**
(21) Application number: 99115880.9
(22) Date of filing: 12.08.1999
(51) Int. Cl.: C07C 251/62, C07C 271/62, A61K 7/40, A61K 7/48

(54) **Oxime carboxylic acid derivatives**
Oximcarbonsäure Derivate
Dérivés d'acides oximinocarboxyliques

(30) Priority: 17.08.1998 EP 98115403
(43) Date of publication of application: 23.02.2000
(73) Proprietor: Givaudan SA, 1214 Vernier-Genève (CH)
(72) Inventor: Anderson, Denise, 8032 Zürich (CH); Frater, Georg, 8400 Winterthur (CH)
(74) Representative: McStea, John Anthony

(56) References cited:
- EP-A- 0 423 874
- DE-A- 1 809 385
- DE-A- 2 227 921
- DE-A- 2 837 204
- DE-A- 3 224 787
- GB-A- 1 048 346
- JP-A- 62 286 961
- US-A- 3 920 752
- US-A- 4 014 915
- DATABASE WPI Section Ch, Week 9633 Derwent Publications Ltd., London, GB; Class B03, AN 96-329447 XP002124668 & JP 08 151356 A (TOKUYAMA SODA KK), 6 November 1996 (1996-11-06)
- DATABASE WPI Week 9536 Derwent Publications Ltd., London, GB; AN 95-272880 XP002124669 & JP 07 173140 A (TOKUYAMA SODA KK)
- GAMALEVICH G.D. ET AL: 'Pheromones of coleoptera.' RUSS. CHEM. BULL. vol. 42, no. 4, 1993, pages 773 - 775

## Description

The present invention relates to oxime carboxylic acid derivatives and the use of oxime carboxylic acid derivatives as precursors for 2,6-dimethyl-hept-5-enal for use as (a) an organoleptic compound, especially for flavours, fragrances and/or (b) a masking agent, and/or (c) an antimicrobial compound.

2,6-dimethyl-hept-5-enal is an aldehyde that is known from "Pheromones of Coleoptra. Synthesis of(+/-)-2.6-dimethyloctyl formate, the biologically active analog of the smaller flour beetle aggregation pheronome" by Gamalevich and Serebryakov, Russ. Chem. Bull., 42, No.4, 773-775 (1993). However, it has not been known as an ingredient in perfumery.

The invention therefore provides the use as a fragrance ingredient of 2,6-dimethyl-oct-5-enal.

The invention further provides a perfume composition comprising 2,6-dimethyl-oct-5-enal.

The invention further provides a method of providing 2,6-dimethyl-oct-5-enal by means of the inclusion of a precursor adapted to generate it under apropriate conditions, the precursor having the Formula I: 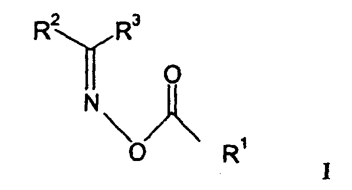 wherein R² and R³ represent any residues of an oxime R²R³ NOH and R¹ represents a radical of the formula

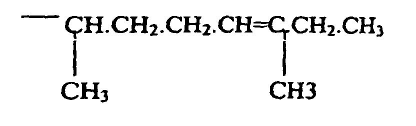

The invention further provides a precursor adapted to provide 2,6-dimethyl-oct-5-enal, the precursor having the Formula I, as hereinabove described.

The invention further provides a method of providing 2,6-dimethyl-oct-5-enal in a desired application, comprising providing in the application a precursor compound having the Formula I as hereinabove defined, such that, in appropriate conditions, the precursor will cleave to provide 2,6-dimethyl-oct-5-enal.

The invention further provides use as precursor for an organoleptic compound, e.g. a fragrance, and/or as a precursor for masking agents, and/or as a precursor for antimicrobial agents of a compound of Formula I.

The compound of Formula I is not limited to any particular stereoisomer, all possible stereoisomers (E/Z isomers, enantiomers, diastereomers) and all mixtures are thus included within the scope of the invention.

Cleavage of the oxime carboxylic acid derivative yields 2,6-dimethyl-oct-5-enal (hereinafter referred to as "the desired aldehyde").

The compound of formula I is nearly odourless at room temperature, atmospheric conditions and 20 to 100 % relative humidity. However, under activating conditions it is cleaved and the desired aldehyde with organoleptic and/or antimicrobial properties is generated..

The activating conditions which lead to cleavage of the precursor and thereby to the liberating of the desired aldehyde may comprise the presence of skin bacteria, especially axilla bacteria, or of an enzyme such as protease or lipase, or elevated temperature or acidic or alkaline pH-values or a combination of at least two of these activating conditions.

A compound of formula I, upon cleavage, provides an oxime and the desired aldehyde having organoleptic and/or antimicrobial and/or masking activities. Therefore a compound of formula I permits the development of useful consumer products with or with enhanced organoleptic and/or antimicrobial and/or masking properties. The aldehyde is useful as a fragrance, masking agent and/or antimicrobial agent. Therefore, the invention relates to the use of a compound of formula I as precursor for an organoleptic compound, e.g. a fragrance, and/or as a precursor for masking agents, and/or as a precursor for antimicrobial agents.

The oxime carboxylic acid derivative of formula I can act as a fragrance precursor in personal care products, in laundry products, cleaning compositions such as all-purpose and hard surface cleaners, pet care products and and environment scents such as air fresheners and candles. It can also act as a precursor for odour masking agents in the same products as the fragrance precursor. It can also act as a precursor for antimicrobial agents for these and further products. The fragrance precursor and the precursor for odour masking agents of the invention may be used individually in an amount effective to enhance or to mask the characteristic odour of a material. More commonly, however, these compounds are mixed with other fragrance components in an amount sufficient to provide the desired odour characteristics. A person skilled in the art will have knowledge how to make best use of the precursor of the invention.

Due to the in situ generation of the active compound, the desired effect is prolonged and the substantivity on different substrates, especially on fabrics, is enhanced. Further, the precursor of the invention provides slow release of the active compounds.

The compound of formula I may preferably be used as a sustained release odorant but also to mask or attenuate undesirable odours or to provide additional odours not initially present in consumer products, i.e. personal care products such as cosmetic products destined for application to human skin such as underarm deodorants or antiperspirants or other deodorants contacting the body, or in hand lotions, hair care products such as shampoos and conditioners, baby powders, baby lotions, ointments, foot products, facial cleansers, body wipes, facial makeup, colognes, after-shave lotions, shaving creams, etc. Additional applications include laundry detergents, fabric softeners, fabric softener sheets, (automatic) dishwasher detergents and all-purpose and hard surface cleaners. Further applications are air fresheners and odorants, candles, odour masking agents and/or antimicrobial agents.

The amount required to produce the desired, overall effect varies depending upon the the product in which it will be used, and the particular effect desired.

For example, depending upon the concentration chosen, when a compound of the formula I is added either singly or as a mixture, e.g. to a deodorant or laundry product composition at levels ranging from 0.1 to 10 % by weight, or most preferred 0.25 to 4 % by weight, an odorant, i.e. one or more odoriferous compounds in an "organoleptically effective amount" is released when the product is used. This newly formed odorant serves to enhance the odour of the product itself or of an fragrance present in the product.

The compound of the formula I can be synthesized in a variety of ways known to those skilled in the art. Convenient methods are outlined in the Examples without limiting the invention thereto.

The present invention is further described by the following examples which are presented solely for the non-limiting purpose of further illustrating the invention.

### Example 1 - Reference example

(a) Octanoic acid 1-bicyclo-[2.2.1]hept-5-en-2-yl-ethanone oxime ester
   To a suspension of 16.2 g sodium caprylate in 200 ml of acetone, 10.5 ml ethyl-chloroformate were dropped in. The mixture was cooled to -5°C and 1 ml pyridine was dropped in. After 40 minutes at -5°C, a solution of 15.12 g 1-bicyclo[2.2.1]hept-5-en-2-yl-ethanone oxime in 100 ml of acetone was dropped in. After stirring for 5.5 hours at room temperature, the mixture was filtered through celite and the filtrate was evaporated to dryness. The residue was diluted with ethyl acetate and washed with water, saturated sodium bicarbonate and water again. The organic phase was dried, filtered and evaporated to dryness. The resulting yellow oil was purified by chromatography to yield 18.6 g of a colourless oil.
   NMR (CDCl₃) δ 6.30-6.08 (m, 1H), 5.98-5.77 (m, 1H), 3.23-3.08 (m, 1H), 3.07-2.93 (m, 1H), 2.93-2.80 (m, 1H), 2.50-2.31 (t, 2H), 1.89 (s, 3H), 1.79-1.57 (m, 2H), 1.56-1.41 (m, 2H), 1.41-1.12 (m, 10H), 1.03-0.78 (m, 3H).
(b) According to the same procedure, octanoic acid 5-methyl-heptan-3-one oxime ester was prepared from 5-methyl-heptan-3-one oxime, sodium caprylate, ethyl-chloroformate and pyridine.
(c) According to the same procedure, benzoic acid 5-methyl-heptan-3-one oxime ester was prepared from 5-methyl-heptan-3-one oxime, sodium benzoate, ethyl-chloroformate and pyridine.

### Example 2 - Reference example

### 4-Oxo-decanoic acid 5-methyl-heptan-3-one oxime ester

A solution of 24.75 g 4-oxo-decanoic acid, 19.07 g 5-methyl-heptan-3-one oxime, 28.56 g N,N'-dicyclohexyl-carbodiimide and 1.68 g 4-pyrrolidinopyridine in 400 ml of 5 dichloromethane was stirred for 24 hours at room temperature. The precipitate was filtered off, the filtrate was diluted with ether, washed with aqueous hydrochloric acid, saturated NaHCO₃ and brine. The organic phase was dried, filtered and evaporated to dryness. The resulting oil-crystal mixture was purified by chromatography to yield 28.75 g of a colourless oil.
NMR (CDCl₃) δ 2.91-2.62 (m, 4H), 2.56-2.01 (m, 6H), 1.85-1.04 (m, 14H),1.03-0.75 (m, 9H) ppm.

### Example 3 - Reference example

(a) -Methyl-heptan-3-one O-(4-allyl-2-methoxy-phenoxycarbonyl) oxime
   A solution of 7.47 g 5-methyl-heptan-3-one oxime and 6.33 g pyridine in 120 ml of dichloromethane was cooled down to 5°C. Then a solution of 13.00 g eugenol-chloroformate in 30 ml of dichloromethane was dropped in and the resulting mixture was stirred for 68 hours at room temperature. Then the mixture was acidified with aqueous hydrochloric acid, extracted with ether, washed with with aqueous hydrochloric acid, saturated NaHCO₃ and brine. The organic phase was dried, filtered and evaporated to dryness. The resulting oil was purified by distillation and chromatography to yield 13.26 g of a colourless oil.
   NMR (CDCl₃) δ 7.25-7.02 (m, 1H), 6.92-6.71 (m, 2H), 6.10-5.83 (m, 1H), 5.30-5.01 (m, 2H), 3.81 (s, 3H), 3.54-3.29 (d, 2H), 2.65-2.27 (m, 4H), 1.90-1.07 (m, 6H), 1.06-0.71 (m, 6H) ppm.
(b) According to the same procedure, 5-methyl-heptan-3-one O-(3-methyl-5-phenyl-oxycarbonyl) oxime was prepared from 5-methyl-heptan-3-one oxime and 3-methyl-5-phenyl-pentanol chloroformate.

### Example 4 - Reference example

Test cloth was washed with a lipase-containing detergent to which the precursor of Example 1a had been added. Headspace analysis of the wet and dry laundry indicated the presence of the decomposition product 1-bicyclo [2.2.1]hept-5-en-2-yl-ethanone oxime which is a fragrance with the common name Terravert. The fragrance level was higher than when the test cloth was washed with a lipase-containing detergent to which an equivalent amount of Terravert had been added.

Analogous experiments were performed with the other precursor compounds mentioned in Examples 1-3. In each of these experiments the result was the same as described before: the fragrance level was always higher using the precursors than when using a detergent containing an equivalent amount of the appropriate fragrance.

### Example 5 - Reference example

Test cloth was washed with a lipase-containing detergent and then a fabric softener, containing one ore more of the precursors of Examples 1-3 was/were added to the rinse cycle. Headspace analysis of the wet and dry laundry indicated the presence of the appropriate odoriferous decomposition products, i.e. the fragrances. The fragrance level was higher than when the test cloth was washed in the same way but then using in the rinse cycle a fabric softener to which an equivalent amount of the corresponding fragrance(s) had been added.

### Example 6 - Reference example

Axilla bacteria cultures, each containing 0.1 % of a precursor from those precursors mentioned in Examples 1-3 were prepared and then incubated for 20 hours at 30°C whereby the precursors were decomposed and yielded inter alia the fragrant decomposition products (oxime, and also alcohol in the cases of Examples 3a and 3b), i.e. fragrances. After filtration from the cells, the presence of the corresponding fragrance was in each case detected by headspace-GC techniques and/or the majority of an 18 member panel.

The same tests were carried out with inactivated cultures and, further, extended to 85°C for 20 minutes. In none of these cases could the odour of the corresponding fragrance be detected after incubation, excluding therefore a hydrolysis by the medium or the culture.

### Example 7 - Reference example

The following set forth examples for the use of the compounds of the present invention in various products. The methods of forming the following compositions are well known to those skilled in the art. All formulations may contain additional ingredients known to those skilled in the art, e.g. colorants, opacifiers, buffers, antioxidants, vitamins, emulsifiers, UV absorbers, silicones and the like. All products can also be buffered to the desired pH. All values are % w/w. "Delayed Release Fragrances" stands in the following for compounds of Examples 1-3.
a) Deo-colognes

| | A % | B % | C % | D % |
|---|---|---|---|---|
| Delayed Release Fragrances | 0.5 | 1.5 | 2.5 | 6.0 |
| Fragrance | 0.5 | 1.5 | 2.5 | 6.0 |
| Triclosan (Ciby Geigy) | 1.0 | - | 0.75 | 1.0 |
| Alcohol to | 100 | 100 | 100 | 100 |

b) Deo-Sticks

| Antiperspirant: | % |
|---|---|
| Ethylene Glycol Monostearate | 7.0 |
| Shea butter | 3.0 |
| Neobee 1053 (PVO International) | 12.0 |
| Generol 122 (Henkel) | 5.0 |
| Kesscowax B (Akzo) | 17.0 |
| Dimethicone Dow Corning 345 | 35.0 |
| Aluminum Sesquichlorhydrate | 20.0 |
| Delayed Release Fragrances | 0.5 |
| Fragrance | 0.5 |

| Antiperspirant: | % |
|---|---|
| Steary Alcohol | 17.0 |
| Castor Wax | 3.0 |
| Talc | 5.0 |
| Aluminum Zirconium Tetrachlorhydrate | 20.0 |
| Delayed Release Fragrances | 1.0 |
| Fragrance | 1.0 |
| Dimethicone Dow 245 | to 100.0 |

| Clear Deodorant Stick: | % |
|---|---|
| Witconol APM | 43.0 |
| Propylene Glycol | 20.0 |
| Alcohol 39C | 20.0 |
| Demin Water | 7.0 |
| Monamid 150ADD | 5.0 |
| Millithix 925 | 2.0 |
| Ottasept Extra | 0.5 |
| Delayed Release Fragrances | 0.75 |
| Fragrance | 0.75 |

| Deodorant Stick: | % |
|---|---|
| Propylene Glycol | 69.0 |
| Demin Water | 21.8 |
| Triclosan | 0.2 |
| Sodium Stearate | 8.0 |
| Delayed Release Fragrances | 0.5 |
| Fragrance | 0.5 |

| Alcohol free Deodorant Stick: | % |
|---|---|
| PPG-3 Myristyl Ether (Witconol APM) | 36.0 |
| Propylene Glycol | 36.0 |
| Demin Water | 19.0 |
| Triclosan | 0.25 |
| Sodium Stearate | 7.75 |
| Delayed Release Fragrances | 0.5 |
| Fragrance | 0.5 |

| Antiperspirant Aerosol: | % |
|---|---|
| Absolute Ethanol | 15.0 |
| Zirconium Aluminum Tetrachlorhydrate | 5.0 |
| Bentone 38 | 1.5 |
| Delayed Release Fragrances | 0.75 |
| Fragrance | 0.75 |
| S-31 Hydrocarbon propellant | to 100.0 |

| Antiperspirant Pump: | % |
|---|---|
| Demin Water | 57.5 |
| Aluminum Sesquichlorhydrate | 20.0 |
| Triton X-102 (Union Carbide) | 2.0 |
| Dimethyl Isosorbide (ICI) | 20.0 |
| Delayed Release Fragrances | 0.25 |
| Fragrance | 0.25 |

| Roll-On: | % |
|---|---|
| Dimethicone DC 354 (Dow Corning) | 69.0 |
| Bentone 38 | 10.0 |
| Rezal 36 GP (Reheis Chem. Co.) | 20.0 |
| Delayed Release Fragrances | 0.5 |
| Fragrance | 0.5 |

In the above examples the following components were used:

| | |
|---|---|
| Triclosan | 5-chloro-2-(2,4-dichlorophenoxy)phenol |
| Neobee 1053 | glycerol tricaprate/caprylate |
| Generol 122 | soya sterol |
| Kesscowax B | cetyl alcohol and glycol polymer |
| Witconol APM | polypropylene glycol-3 myristyl ether |
| Monamid 150 ADD | cocoamide diethanolamine |
| Millithix 925 | dibenzylidene sorbitol |
| Ottasept Extra | quatemium 18 hectorite |
| Bentone 38 | quatemium 18 hectorite |
| Triton X-102 | octoxynol-13 |
| Dimethicone DC 354 | mixture of fully methylated linear siloxanepolymers end-blocked with trimethylsiloxy units |
| Rezal 36 GP | Aluminum zirconium tetrachlorohydrexglycine |

Applying to the human skin all these malodour preventing products showed a more intense and longer lasting effect than equivalent products having added the same amount of the corresponding fragrances which result from the decomposition of the Delayed Release Fragrances".

### Example 8 - Reference example

The following two examples exemplify the production of softeners containing at least one of the precursors of examples 1-3 yielding delayed release fragrances as decomposition products. These softeners have the advantages as already explained in Example 5.
a) Fabric softener of the ester quat type (4 x concentrate):

| ingredients | chemical name | % |
|---|---|---|
| | | |

| PHASE A | | |
|---|---|---|
| deionised water | | to 100.0 |
| MgCl₂ (saturated sol.) | Magnesium chloride | 1.0 |

| PHASE B | | |
|---|---|---|
| Rewoquat WE 18 | Di-(tallowcarboxyethyl)hydroxy ethyl methylammonium methosulfate | 15.0 |
| Genapol O 100 | Ethoxylated fatty alcohol C16-C18 10EO | 2.0 |
| antifoam DB 31 | | 0.5 |

| PHASE C | | |
|---|---|---|
| isopropyl alcohol | | 3.0 |
| preservative | | Qs |
| precursor and perfume | | Qs |

preparation process:
While stirring and heating to 65° C part A was admixed, then part B which had been preheated to 65° C. After cooling to room temperature part C was added.
The pH value of the finished product was 2.60. The recommended level of the precursor and perfume is 1.0 %. Delayed release fragrances of Examples 1-3 could be any part of this 1.0%.
b) Fabric softener of the ester quat type (1 x concentrate):

| ingredients | chemical name | % |
|---|---|---|
| | | |

| PHASE A | | |
|---|---|---|
| deionised water | | to 100.0 |

| PHASE B | | |
|---|---|---|
| Rewoquat WE 18 | Di-(tallowcarboxyethyl)hydroxy ethyl methylammoniummethosulfate | 6.0 |
| Dobanol 25-9 | Ethoxylated fatty alcohol C12-C15 9EO | 0.50 |
| antifoam DB 31 | | 0.10 |

| PHASE C | | |
|---|---|---|
| Myacide BT 30 | 2-bromo-2-nitropropane 1,3 diol | 0.03 |
| Proxel GXL | Benzisothiazolinone sodium salt | 0.02 |
| precursor and perfume | | Qs |

preparation process:
While stirring and heating to 65° C, part A was admixed, then part B which had been preheated to 65° C. After cooling to room temperature part C was added.
The pH value of the finished product was 3.5. The recommended level of the precursor and perfume is 0.3 % and delayed release fragrances of Examples 1-3 could be any part of this 0.3 %.

## Claims

1. Use as a fragrance ingredient of 2,6-dimethyl-oct-5-enal.

2. A precursor adapted to provide 2.6-dimethyl-oct-5-enal, the precursor having the Formula I: 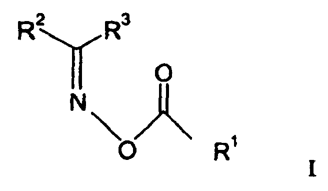 wherein R² and R³ represent any residues of an oxime R²R³ NOH and R¹ represents a radical of the formula

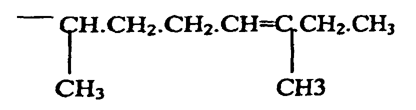

3. A method of providing 2,6-dimetllyl-act-5-enal in a desired application, comprising providing in the application a precursor compound having the Formula I: 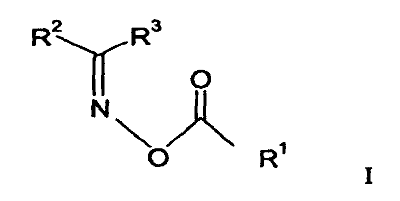 wherein R² and R³ represent any residues of an oxime R²R³ NOH and R¹ represents a radical of the formula 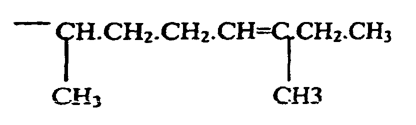 such that, in appropriate conditions, the precursor will cleave to provide 2,6-dimethyl-oct-5-enal.

4. Use as precursor for an organoleptic compound, and/or as a precursor for masking agents, of a compound of Formula I as defined in claim 2.

5. Use of a compound of formula (I) as defined in claim 2 for the production of an antimicrobial agent.

## Patentansprüche

1. Verwendung von 2,6-Dimethyl-oct-5-enal als Duftstoffbestandteil.

2. Vorläuferverbindung, die dazu eingerichtet ist, 2,6-Dimethyl-oct-5-enal bereitzustellen, wobei die Vorläuferverbindung die Formel I aufweist: 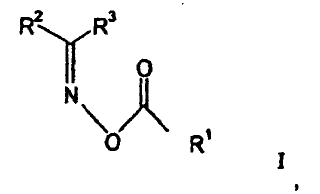 worin R² und R³ für beliebige Reste von einem Oxim R²R³NOH stehen, und R¹ für einen Rest der Formel 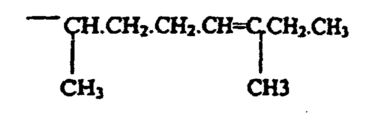 steht.

3. Verfahren zur Bereitstellung von 2,6-Dimethyl-oct-5-enal bei einer gewünschten Anwendung, das umfasst ein Bereitstellen einer Vorläuferverbindung, die die Formel I aufweist, bei der Anwendung: 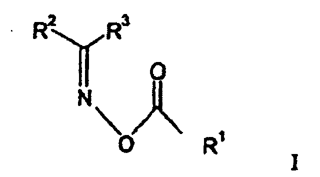 worin R² und R³ für beliebige Reste von einem Oxim R²R³NOH stehen, und R¹ für einen Rest der Formel: 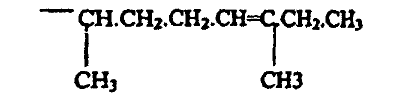 steht, so dass sich die Vorläuferverbindung bei geeigneten Bedingungen spaltet, um 2,6-Dimethyl-oct-5-enal bereitzustellen.

4. Verwendung von einer Verbindung der Formel I, wie in Anspruch 2 definiert, als Vorläuferverbindung für eine organoleptische Verbindung und/oder als eine Vorläuferverbindung,für Maskierungsmittel.

5. Verwendung von einer Verbindung der Formel I, wie in Anspruch 2 definiert, für die Herstellung eines antimikrobiellen Mittels.

## Revendications

1. Utilisation de 2,6-diméthyl-oct-5-énal comme ingrédient de parfum.

2. Précurseur adapté pour produire du 2,6-diméthyl-oct-5-énal, le précurseur ayant la formule I : 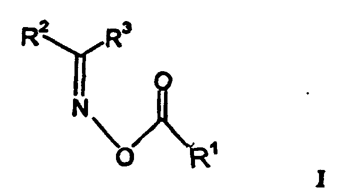 dans laquelle R² et R³ représentent tout résidu d'un oxime R²R³NOH et R¹ représente un radical de formule :

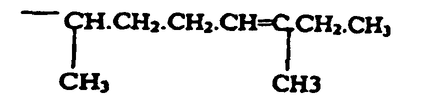

3. Procédé d'obtention de 2,6-diméthyl-oct-5-énal dans une application souhaitée, comprenant l'apport dans l'application d'un composé précurseur de formule I : 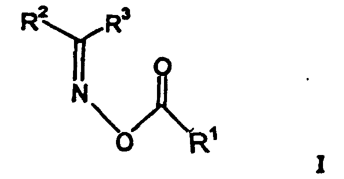 dans laquelle R² et R³ représentent tout résidu d'un oxime R²R³NOH et R¹ représente un radical de formule : 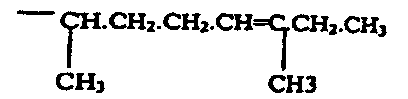 de façon telle que, dans des conditions appropriées, le précurseur est clivé en produisant du 2,6-diméthyloct-5-énal.

4. Utilisation d'un composé de formule I tel que défini dans la revendication 2, comme précurseur d'un composé organoleptique et/ou comme précurseur d'agents de masquage.

5. Utilisation d'un composé de formule (I) tel que défini dans la revendication 2, pour la production d'un agent antimicrobien.
